(19) Europäisches Patentamt
European Patent Office
Office européen des brevets

(11) Publication number: **0 428 000 A1**

(12) **EUROPEAN PATENT APPLICATION**

(21) Application number: **90120724.1**

(22) Date of filing: **29.10.90**

(51) Int. Cl.[5]: **C12Q 1/37**, G01N 33/533, G01N 33/58, C07K 7/06

(30) Priority: **03.11.89 US 431501**

(43) Date of publication of application: **22.05.91 Bulletin 91/21**

(84) Designated Contracting States: **DE ES FR IT**

(71) Applicant: **ABBOTT LABORATORIES**
**CHAD-0377, AP6D/2, One Abbott Park Road**
**Abbott Park, Illinois 60064-3500(US)**

(72) Inventor: **Krafft, Grant A.**
**2433 Saranac Court**
**Glenview, Illinois 60025(US)**
Inventor: **Wang, Gary T.**
**9148 Grosspoint Road**
**Skokie, Illinois 60077(US)**
Inventor: **Matayoshi, Edmund D.**
**4410 Hillshire Drive**
**Richmond, Illinois 60071(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Fluorogenic substrates for the detection of proteolytic enzyme activity.**

(57) In the present invention, continuous data acquisition of substrate hydrolysis by a protease enzyme is achieved through the use of a fluorescent peptide substrate, wherein the quantum yield of fluorescence increases upon proteolytic cleavage of the substrate. Incubation of a viral protease enzyme with the fluorogenic substrate results in a time-dependent increase in fluorescence intensity which is linearly related to the extent of substrate hydrolysis.

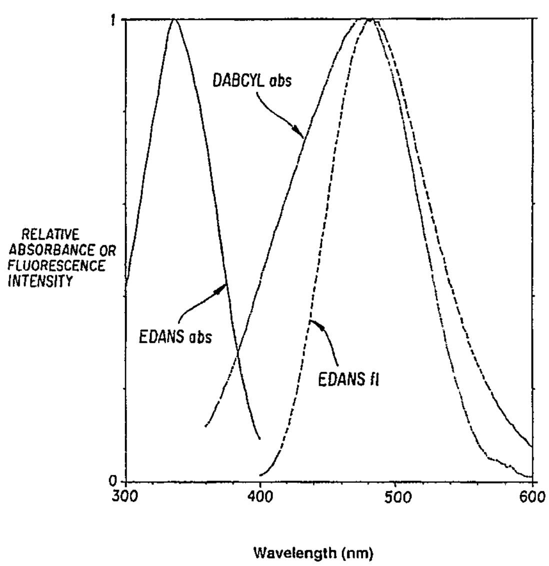


FIG. 1

EP 0 428 000 A1

# FLUOROGENLC SUBSTRATES FOR THE DETECTION OF PROTEOLYTIC ENZYME ACTIVITY

## REFERENCE TO GOVERNMENT GRANT

The invention described herein was made with Government support under Contract Number AI27220-01, awarded by the National Institute of Allergy and Infectious Diseases. The Government has certain rights to this invention.

## BACKGROUND OF THE INVENTION

### 1. Field of the Invention

The present invention relates to the field of the detection and measurement of proteolytic enzyme activity. In particular, the invention relates to fluorogenic assays for the detection and measurement of proteolytic enzyme activity, and the fluorogenic substrates therefor.

### 2. Description of Related Art

Retroviruses constitute a family of single-stranded RNA-containing viruses that cause a variety of diseases in animals and man. [R. Weiss, et al., RNA Tumor Viruses (Cold Spring Harbor Laboratory Press, Cold Spring Harbor, NY, 1985.)] These viruses replicate via a DNA intermediate which is synthesized by a viral-encoded reverse transcriptase. As with other classes of positive-strand RNA viruses, retroviral proteins are initially synthesized as large polyprotein precursors which are later processed by posttranslational cleavage. The internal structural proteins, replicative enzymes, and envelope glycoproteins of retroviruses are encoded by the gag, pol and env genes, respectively. Translation of the polycistronic viral messenger RNA (mRNA) results in the synthesis of two precursor polyproteins: $Pr^{gag}$, which contains the structural capsid proteins, and $Pr^{gag\text{-}pol}$, a read-through protein that contains both the structural proteins and the replicative enzymes. The env gene products are translated as a precursor polyprotein from a separately spliced mRNA transcript. Retroviruses also encode a small, 10-12 kilodalton (kd) protease which is generally expressed as part of the $Pr^{gag\text{-}pol}$ precursor, except in the case of the avian retroviruses, where it is synthesized as the C-terminal portion of $Pr^{gag}$. [S. Oroszlan & T. Copeland, Curr. Top. Microbiol. Immunol. 115, 221(1985)].

Retroviral proteases are responsible for the processing of both $Pr^{gag}$ and $Pr^{gag\text{-}pol}$ precursor polyproteins at specific cleavage sites. Cleavage is believed to occur during or after virion assembly and has been shown, in the case of the human immunodeficiency virus (HIV) and murine leukemia virus, to be required for the maturation of infectious virus particles. [S. Crawford, et al., J. Virol. 53, 899 (1985); I. Katoh, et al., Virology 145, 284 (1985); N.E. Kohl et al., Proc. Natl. Acad. Sci. U.S.A. 85, 4684 (1988).] Thus in regard to AIDS, the inhibition of HIV protease activity has become an important goal for antiviral drug design efforts, and the detection of proteolytic activity is concomitantly important for monitoring drug therapy and diagnosing disease.

A variety of techniques have been previously employed to measure retroviral proteolytic activity, including analysis of the gag polyprotein and its cleavage products by Western blot [I. Katoh, et al., Nature 329, 654 (1987); S. Seelmeier, et al., Proc. Natl. Acad. Sci. 85, 6612 (1988); C.Z. Giam, et al., J. Biol. Chem. 263, 14617 (1988); J. Schneider, et al., Cell 54, 363 (1988); J. Hansen, et al., EMBO J. 7, 1785 (1988); M.C. Graves, et al., Proc. Natl. Acad. Sci. 85, 2449 (1988)], high performance liquid chromatography (HPLC) [S. Billich, et al., J. Biol. Chem. 263, 17905 (1988); P.L. Darke, et al., J. Biol. Chem. 264, 2307 (1989); P.L. Darke, et al., Biochem. Biophys. Res. Comm. 156, 297 (1988); A.D. Richards, et al., FEBS Lett 247, 113 (1989); T.D. Meek, et al., Proc. Natl. Acad. Sci. 86, 1841(1989); R.F. Nutt, et al., Proc. Natl. Acad. Sci. 85, 7129 (1988); H.G. Krausslich, et al., Proc. Natl. Acad. Sci. 86, 807 (1989); M.L. Moore, et al., Biochem. Biophys. Res. Comm. 159, 420 (1989)], or thin-layer electrophoretic analysis of synthetic peptide cleavage fragments [M. Kotler, et al., Proc. Natl. Acad. Sci. 85, 4185 (1988); M. Kotler, et al., J. Biol. Chem. 264, 3428 (1989).] Each of these methods are relatively time-consuming and impractical for screening and characterizing large numbers of protease inhibitors. These methods are also lacking in precision for enzymological studies because they do not allow for the continuous measurement of the protease reaction kinetics.

The feasibility of applying intramolecular resonance energy transfer to the measurement of hydrolase activity was demonstrated nearly two decades ago. [S.A. Latt, et al., Anal. Biochem. 50, 56 (1972); A. Carmel, et al., FEBS Lett 30, 11(1973)] Since then, however, the use of the methodology (or analogous quenching/cleavage strategies) for protease assay design has seen limited practical application [A. Yaron, et al., Anal. Biochem. 95, 228 (1979) and references therein; A. Persson, et al., Anal. Bicchem. 83, 2,96 (1977); N. Nishino, et al., J. Biol. Chem. 255, 3482 (1980); C. Deyrup, et al., Anal. Biochem. 129, 502 (1983); I. Yu. Filippova, et al., Bioorg. Khim. 12, 1172 (1986); J. Pohl, et al., Anal. Biochem. 165, 96 (1987); S.J. Pollack, et al., J. Am. Chem. Soc. 111, 5961 (1989).]

## SUMMARY OF THE INVENTION

The present invention involves a novel fluorogenic substrate made from a fluorescent donor and a quenching acceptor attached to a peptide, wherein the peptide has a configuration sufficient to enable intramolecular resonance energy transfer between the fluorescent donor and the quenching acceptor, and wherein the peptide can be cleaved by a viral protease enzyme. Typically the peptide separates the fluorescent donor and the quenching acceptor at a distance of less than about 100 angstroms. The fluorescent donor can be attached to the peptide's C-terminus, and the quenching acceptor can be attached to the peptide's N-terminus, or visa versa.

The novel substrates can be advantageously used in assays for detecting the presence or activity of a viral protease enzyme in a test sample. In such an assay, the test sample is combined with a fluorogenic substrate of the present invention, and if the enzyme is present in the test sample then the enzyme cleaves the substrate, thereby separating the fluorescent donor and the quenching acceptor. The resultant fluorescent emission can then be detected or measured.

The substrates of the present invention can also be advantageously used to detect the activity of a protease enzyme inhibitor. In such an assay, the inhibitor, a protease enzyme and a fluorogenic substrate are combined. The effectiveness of the enzyme inhibitor is indicated by the extent to which the enzyme is inhibited from hydrolyzing the substrate.

The novel substrates enable the continuous monitoring of the donor's fluorescent emission caused by substrate hydrolysis. Because of their simplicity and precision in the determination of reaction rates required for kinetic analysis, these assays offer many advantages over conventional assays using high performance liquid chromatography or electrophoresis techniques. The substrates have been especially useful in detecting the presence or inhibition of human immunodeficiency virus protease and avian myeloblastosis virus (AMV) protease.

## BRIEF DESCRIPTION OF THE DRAWINGS

FIG. 1 depicts the absorption spectrum and the technical fluorescence spectrum of 5-((2-aminoethyl)-amino)naphthalene-1-sulfonic acid (EDANS), and the absorption spectrum of 4-(4-dimethylaminophenyl) azobenzoic acid (DABCYL).

FIG. 2 depicts the chromatograms for fluorescence and 475 nanometers absorbance of the fluorogenic substrate DABCYL-gaba-Ser-Gln-Asn-Tyr-Pro-Ile-Val-Gln-EDANS (Substrate I), prior to addition of HIV protease.

FIG. 3 depicts the chromatograms for fluorescence and 475 nanometers absorbance of the products of Substrate I following hydrolysis by HIV protease.

FIG. 4 depicts the inhibition of HIV protease activity by pepstatin in the form of a Dixon plot of pepstatin concentration vs. inverse of reaction velocity.

FIG. 5 depicts the inhibition of HIV protease activity by pepstatin in the form of a Cornish-Bowden plot of pepstatin concentration vs. substrate concentration/reaction velocity.

## DETAILED DESCRIPTION OF THE INVENTION

The 11 kd protease enzyme, which is encoded by the human immunodeficiency virus-1 (HIV-1), is required for the processing of viral polyproteins and for the subsequent maturation of the infectious virus. Therefore, the inhibition of protease activity is a major target for the therapeutic treatment of AIDS. To facilitate in-depth studies on the enzymology and inhibition of HIV protease, the present invention describes a novel assay based upon intramolecular fluorescence energy transfer.

The present invention provides novel fluorescence assays for retroviral proteases, such as those encoded by HIV and the Rous sarcoma virus-related AMV. The assays use novel fluorogenic protease substrates which include a peptide structure that combines a fluorescent donor or fluorophore with a quenching acceptor. Generally, the fluorescent donor and quenching acceptor are attached at the C- and N-peptide termini, respectively. Suitable fluorescent donors for the present invention include, but are not limited to, fluorescein and fluorescein derivatives, N-(2-aminopentyl)-3-amino-2,7-disulfo-1,8-naphthalimide dipotassium salt (lucifer yellow) and derivatives thereof, 5-((2-aminoethyl)amino)naphthalene-1-sulfonic acid (EDANS), coumarin and coumarin derivatives, and equivalents thereof. Suitable quenching acceptors for the present invention include, but are not limited to, 2,4-dinitrophenyl (DNP), 4-(4-dimethylaminophenyl) azobenzene sulfonic acid (DABCYL), 4-(4-dimethylaminophenyl) azobenzoic acid (DABCYL) and equivalents thereof. While virtually any fluorophore and quenching acceptor can be used in the present invention, the specific donor/acceptor combination used to make a given fluorogenic protease substrate is chosen for its optimal energy transfer efficiency.

The peptide joins the donor/acceptor pair such that the intrinsic fluorescence of the donor, e.g., EDANS, is dramatically reduced due to intramolecular resonance energy transfer between the fluorescent donor and the quenching acceptor, e.g., DABCYL. Because intramolecular resonance energy transfer becomes insignificant at distances beyond about 100 angstroms, the full fluorescence quantum yield of the fluorophore is restored after the peptide structure is cleaved by an enzyme, such as a protease, thereby liberating the fluorescence quenching acceptor-peptide fragment from the fluorescence donor-peptide fragment of the original substrate. The peptide structure which combines the donor/acceptor combination can be any peptide which provides both the necessary distance between the donor and acceptor and an appropriate cleavage site for the protease of interest.

Specific examples of fluorogenic protease substrates, for the continuous detection or measurement of a protease reaction, include the following:

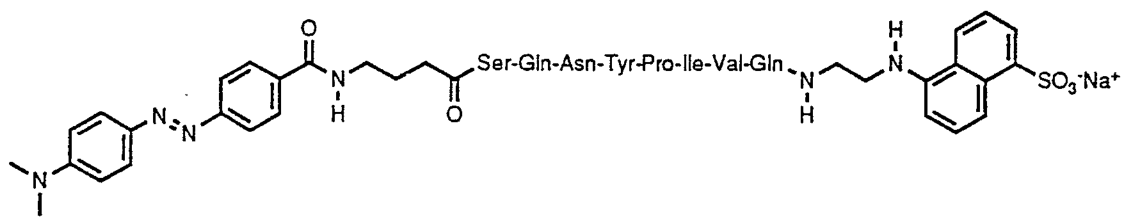

DABCYL **Substrate I** EDANS

and

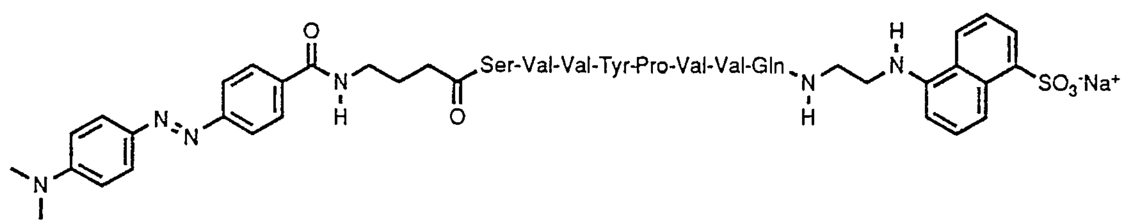

DABCYL **Substrate II** EDANS

The peptide sequence of Substrate I is an octapeptide, Ser-Gln-Asn-Tyr-Pro-Ile-Val-Gln, which corresponds to the naturally occurring gag p17/gag p24 cleavage site for HIV protease which cleaves the peptide at the Tyr-Pro peptide bond. The peptide sequence used in Substrate II, Ser-Val-Val-Tyr-Pro-Val-Val-Gln, was designed to take advantage of possible symmetry in the active site of HIV protease. However, the sequence was found to be a better substrate for AMV protease. Although not a natural sequence for AMV protease, a high Val content is predicted to enhance substrate efficiency based on an examination of

the various cleavage sites on AMV polyprotein [T.D. Copeland and S. Oroszlan, in Peptides: Synthesis, Structure and Function, D.H. Rich and E. Gross, Eds. (Pierce, Rockford, IL, 1982) pp. 497-500.] A gamma-aminobutyric acid (gaba) spacer was inserted between the DABCYL group and the N-terminal Ser to avoid potential steric hindrance of substrate binding by the bulky acceptor.

With the substrates of the present invention, an enzyme's proteolytic activity can be monitored over time due to the continuous increase in fluorescence intensity; as the enzyme cleaves the peptide there is a continuous and increasing liberation of the fluorophore. Furthermore, this increase is linearly related to the rate of hydrolysis of the fluorogenic substrate because for each mole of substrate which is cleaved, the total fluorescence is increased by the net intensity due to the presence of one mole of newly created donor-peptide fragment. This phenomenon has been found to advantageously serve as the basis of a sensitive and quantitative assay in which proteolytic reaction velocities can be determined by detecting the change in fluorescence over time. In addition, the fluorogenic substrates of the present invention can be used to determine an enzyme's activity in the presence of an enzyme inhibitor. Therefore, the novel substrates can be advantageously used for the rapid and large-scale screening of compounds derived from a variety of sources, including existing libraries of proteolytic enzyme inhibitors and microbial fermentation beers, as well as new synthetic enzyme inhibitors.

In brief, the inhibitory potency of a compound can be determined by the following method. A reaction mixture is formed by combining a protease enzyme, (e.g., HIV protease), a protease inhibitor compound, including but not limited to pepstatin, acetylpepstatin, etc., and a fluorogenic substrate (e.g., Substrate I) of the present invention. Typically, the assay reaction is performed using buffered solutions, and the reaction is allowed to proceed to virtual completion. The hydrolysis of the fluorogenic substrate by the protease enzyme is monitored by steady state fluorescence using a spectrofluorometer. Fluorescence intensity measurements can be acquired continuously and recorded directly by an interfaced microcomputer. The rate of fluorescent substrate hydrolysis can then be computed from a linear regression analysis. In this assay, the rate of hydrolysis of the fluorogenic substrate is directly proportional to the concentration of the protease. By omitting the protease inhibitor compound from the reaction mixture, the rate of change in fluorescent intensity due to the enzyme's activity can be determined. Virtually any protease enzyme can be detected by the present invention. The protease enzymes used in the exemplary descriptions which follow can be produced in accordance with the descriptions of J. Hansen, et al., EMBO J. 7, 1785 (1988); T.D. Meek et al., Proc. Natl. Acad. Sci. 86, 1841 (1989); and R.F. Nutt et al., Proc. Natl. Acad. Sci. 85, 7129 (1988) which are incorporated by reference herein.

The success of the fluorescence intramolecular resonance energy transfer approach is based upon the choice of the appropriate donor/acceptor energy transfer pair. By optimizing the energy transfer efficiency between the donor and acceptor, the residual fluorescence of the donor component of the intact substrate is minimized, which in turn results in a large change in fluorescence signal as the substrate is cleaved. This characteristic enables reaction rate determinations from the hydrolysis of a very small fraction of total substrate, an aspect that is highly desirable for estimating initial reaction velocities.

The selection of EDANS and DABCYL as one preferred donor/acceptor pair was based upon a number of considerations. First, there is an extremely high spectral overlap of the EDANS fluorescence emission with the strong visible absorption band of DABCYL, leading to very efficient energy transfer.

Second, the use of a relatively long lived fluorescence donor, such as EDANS, leads to improved suppression of residual substrate fluorescence, because enhancement of intramolecular resonance energy transfer due to diffusion of the donor and acceptor moieties becomes significant. For example, in Substrates I and II the maximal donor/acceptor separation distance (R) is expected to be about 29 angstroms (assuming an extended octapeptide configuration). $R_0$, the Foerster distance for 50% energy transfer efficiency, was estimated to be 33 angstroms for this donor/acceptor pair. Therefore, R is less than $R_0$, and the efficiency of energy transfer increases steeply with decreasing R for distances $R < R_0$. Assuming a relative intramolecular diffusion coefficient of the ends of Substrate I to be $5 \times 10^{-7}$ $cm^2$/second [E. Haas, et al., Biopolymers 17, 11 (1978)), a 20-fold quenching of donor fluorescence would be achieved at $R = 0.61 R_0$, and a 65-fold quenching at $R = 0.5 R_0$. The donor and acceptor could move approximately 13 angstroms relative to one another during the excited state lifetime of EDANS.

$R_0$ was estimated using a value of 0.67 for the orientation factor, which is valid when donor and acceptor dipoles undergo a dynamic randomization of relative orientations during the excited state lifetime of the donor [L. Stryer, Ann. Rev. Biochem. 47, 819 (1978) for a review of resonance energy transfer calculations.] This assumption was considered reasonable because of the considerable flexibility of the DABCYL and EDANS linkages to the peptide, as well as the flexibility of the peptide itself. A value for the donor quantum yield (Q) of 0.13 was used on the basis of the yield measured for the substrate fragment Pro-Ile-Val-Gln-EDANS, made by comparison to quinine sulfate in 1 N sulfuric acid [Q= 0.546; ,J.N.

Demas, et al., J. Phys. Chem. 75, 991(1971).]

Third, the detectability of EDANS is enhanced by several factors, including its moderately good quantum yield, reasonable stability against photobleaching, and a large spectral separation of its absorption and emission bands. Figure 1 illustrates the absorption spectrum and the technical fluorescence spectrum of EDANS, as well as the DABCYL absorption spectrum. The figure illustrates the overlap of the EDANS fluorescence and the DABCYL absorption spectra, which leads to their efficient rate of energy transfer as a donor/acceptor pair. The absorption spectrum of EDANS is included to show the large spectral separation of its absorption and emission bands (Stokes shift of over 100 nanometers), which resulted in the ability to detect low concentrations of EDANS, and therefore, further enhanced the sensitivity of the assay.

Fourth, the solubility of EDANS in water is particularly beneficial for solubilizing hydrophobic peptides such as those which are suitable for use in fluorogenic substrates for HIV protease.

Fifth, the well-separated visible absorption band of DABCYL facilitates substrate purification and quantitation, as well as the analysis of the substrate fragments produced by proteolysis. The fact that DABCYL is also non-fluorescent improves the detectability of EDANS fluorescence because it allows the latter to be measured using cut-off filters or very wide band pass monochromator slits or filters. In principle it should be possible to utilize an acceptor which is also fluorescent and to obtain reaction rates by monitoring the time-dependent decrease in sensitized acceptor fluorescence. In practice, however, obtaining comparable sensitivity by use of this method is more difficult because it requires using a donor/acceptor pair in which (1) the donor can be excited without significant direct excitation of the quenching acceptor, and (2) the acceptor and donor fluorescence spectra are well-separated from one another.

In the present invention, intramolecular resonance energy transfer can be effectively used to assay retroviral protease activity even when relatively large peptides are used in the fluorescent substrates. The simplicity, rapidity, and precision of the intramolecular resonance energy transfer assay facilitates its adaptation to many uses and formats. The substrates are especially useful for enzymological studies and for the characterization of compounds which may be of value as protease inhibitors. For large scale screening, the intramolecular resonance energy transfer assay lends itself readily to automated formats such as the 96-well fluorescence plate reader. The intramolecular resonance energy transfer approach is also useful in studies on the substrate specificity requirements of the retroviral proteases, because an efficient donor/acceptor pair can be employed with virtually any peptide sequence or macromolecule, requiring only that the donor and acceptor groups can be positioned so as to minimally perturb the enzyme/substrate interaction.

The invention is further illustrated by the following examples and experiments.

## EXAMPLES

### Example 1

### Preparation of Fluorogenic Substrates

The following example describes the preparation of fluorogenic Substrate I. The fluorescent donor was EDANS, and the quenching acceptor was DABCYL linked via a gaba spacer.

The Ser-Gln-Asn-Tyr-Pro-Ile-Val-Gln and Ser-Val-Val-Tyr-Pro-Val-Val-Gln octapeptides (Multiple Peptide Systems, San Diego, CA) of Substrates I and II were derivatized using conventional condensation chemistry of DABCYL and EDANS with the octapeptides' amino and carboxyl termini, respectively.

a Preparation of DABCYL-gaba-N-hydroxy-succinimide

4-(p-Dimethylaminophenyl-azo)-benzoic acid (dabcyl-OH), prepared by adding a sufficient amount of pH 4.0 buffer to a solution of the corresponding sodium salt (available from Sigma, St. Louis, MO) in boiling water, was dissolved in dimethyl formaldehyde (DMF), and 1.3 equivalents each of dicyclohexyl carbodiimide (DCC) and N-hydroxysuccinimide (NHS) were added. After completion of the reaction, as

indicated by thin layer chromatography (TLC), the mixture was concentrated and the residue was triturated with methylene chloride to remove insoluble by-product, N, N′-dicyclohexylurea (DCU). The methylene chloride solution was concentrated to provide almost pure DABCYL-NHS. This material was redissolved in DMF, and 1.2 equivalents of 4-amino-butyric acid (gaba) were added, followed by the addition of 1.2 equivalents of triethylamine. The mixture was stirred at room temperature until completion of the reaction (as indicated by TLC). The DMF was evaporated, and the residue was recrystallized from methanol-methylene chloride mixture to provide substantially pure DABCYL-gaba-OH.

DABCYL-gaba-OH (2.0 g, 5.6 mmol.) was dissolved in dry DMF (300 mL). To the solution was added 0.8 grams of NHS (1.2 eq) and 1.4 grams of DCC (1.2 eq). TLC indicated completion of the reaction after stirring at room temperature overnight. The solid material was filtered. The filtrate was concentrated, and the residue was washed, twice, with methylene chloride to remove remaining DCU. Then, the methylene chloride solution was chromatographed on a silica column, with EtOAc as the elutant, to provide 1.58 grams of pure DABCYL-gaba-NHS.

b. Preparation of DABCYL-gaba-Ser-Gln-Asn-Tyr-Pro-Ile-Val-Gln

The peptide Ser-Gln-Asn-Tyr-Pro-Ile-Val-Gln (49.0 mg, 0.046 mmol) was dissolved in dry DMF (50 mL), and triethylamine (approximately 0.2 mL, excess) was added to the solution. Approximately 1.2 equivalents of DABCYL-gaba-NHS was then added to the solution. After stirring at room temperature overnight, the solvent was evaporated and the residue was triturated with methylene chloride. Substantially pure product (60 mg) was obtained as a dark red solid upon filtration.

c. Preparation of DABCYL-aaba-Ser-Gln-Asn-Tyr-Pro-Ile-Val-Gln-EDANS

DABCYL-gaba-Ser-Gln-Asn-Tyr-Pro-Ile-Val-Gln (50.0 mg, 0.039 mmol) was dissolved in dry DMF (50 mL). To the solution was added 1.5 equivalents of 5-(2-aminoethylamino)-1 -naphthlenesulfonic acid sodium salt (EDANS, Sigma), 1.5 equivalents of NHS, and 1.5 equivalents of 1-ethyl-3-(3-dimethylaminopropyl)-carbodiimide hydrochloride (EDC; Sigma). After stirring at room temperature overnight, the mixture was concentrated, and the residue was redissolved in a dimethyl sulfoxide (DMSO)-water mixture and subjected to HPLC purification.

HPLC conditions included the following: (i) Rainin C-8 reverse phase column (21 X 250 mm); and (ii) a solvent gradient using solvent A (100% water with 10 mM sodium acetate [NaOAc], pH 5) for 10 minutes, then linear gradient to 60% solvent B (90% aqueous acetonitrile with 10 mM NaOAc, pH 5) over a period of 60 minutes. Typically, when a dual-channel UV-VIS detector was used, the desired HPLC peak had a ratio of absorption at 490 nm to absorption at 260 nm of roughly one, which is diagnostic for the desired product. The fractions that contained the desired product were combined and concentrated. The residue was treated with DMF and filtered to remove sodium acetate. The DMF filtrate was then evaporated to dryness, and the residue was treated with methylene chloride and hexane. A crystalline dark red solid (49.0 mg) was obtained upon filtration.

Example 2

Fluorogenic Assay for Screening Inhibitors of HIV Protease

The inhibitor compound (pepstatin, Sigma) was dissolved in DMSO, and a small aliquot was further diluted with DMSO to 30 times the final concentration desired for testing. The inhibitor solution (20 µL) was slowly added to a buffer solution (380 µL; 0.1 M NaOAc, 1 M sodium chloride, 1 mM ethylenediamine tetraacetate, 1 mM dithiothreitol and 1.0 mg/ml bovine serum albumin [BSA]) while vigorously vortexing, to yield an aqueous solution of the inhibitor. The buffered inhibitor solution was stored at room temperature, and it was made within a few hours of use, to minimize time-dependent aggregation of poorly water-soluble compounds.

The protease enzyme, HIV protease, was diluted with the buffer solution which further contained 10%

DMSO to form a protease enzyme solution (6.7 nM; 73 ng/mL). The protease enzyme solution was stored on ice.

A fluorogenic Substrate I solution (24 μM; DABCYL-gaba-Ser-Gln-Asn-Tyr-Pro-Ile-Val-Gln-EDANS) was prepared by diluting the substrate (480 μM in pure DMSO) 20-fold with buffer while vigorously vortexing. The substrate solution was also stored at room temperature.

The assay reaction was carried out in a fluorescence microcell (3 mm), for which a total sample volume of 120 microliters is adequate in most fluorometers. The enzyme solution (20 μL) was mixed with the inhibitor solution (80 μL) directly in the microcell. The microcell was placed in the fluorometer cell holder, at 30°C for at least one minute. Prewarmed substrate solution (20 μL at 30°C) was then added to the microcell, and the fluorescence intensity of the reaction mixture was recorded as a function of time. The first two minutes of data were ignored to ensure complete temperature equilibration of the reaction mixture. The rate of change of the fluorescence intensity over the subsequent five to eight minutes was determined by linear regression. The observed rate was directly proportional to the moles of substrate cleaved per unit time. Therefore, the per cent inhibition was calculated as 100 x (1 - (rate in presence of inhibitor)/(rate in absence of inhibitor)).

## Example 3

### Fluorogenic Assays for Protease Enzymes

Rate measurements of the change of the fluorescence intensity over time were also performed for reaction mixtures containing a protease enzyme and a fluorogenic substrate of the present invention. The assays were performed substantially in accordance with the procedure described in Example 2, with the exception that the inhibitor compound was omitted.

## Example 4

### HPLC Analysis of the Fluorogenic Substrates

The following example describes the HPLC analysis of the novel fluorogenic substrates and their reaction products.

Analytical HPLC and the isolation of substrate fragments were performed in accordance with methods well known in the art on a Hewlett Packard 1090M system equipped with diode array absorbance and fluorescence detectors, using either a Brownlee 250 x 4.6 mm C8 silica column at pH values below 5, or a Hamilton 50 x 4.1 mm PRP3 polymer column over a pH range of 2-8. A water to acetonitrile gradient was used; either 0.1% trifluoroacetic acid (pH of about 2), 10 mM sodium acetate (pH 4.9), or 10 mM Tris-HCl (pH 8) was included to vary the pH.

The detection system permitted the simultaneous acquisition of the chromatograms for fluorescence and several absorbance wavelengths, as well as the absorption spectra for all peaks. The fluorescence (excitation at 340 nm, emission at 490 nm) and 475 nm absorbance signals, which arise respectively from the EDANS and DABCYL groups, were used to monitor and quantitate the fragments produced by proteolysis of the substrate.

The chromatograms for fluorescence and 475 nm absorbance are overlayed in Figures 2 and 3: (Fig.2) fluorogenic Substrate I, prior to addition of HIV protease; and (Fig. 3) products of Substrate I following hydrolysis by HIV protease for 2.5 hours under the conditions described in Example 2.

The molar fluorescence enhancement of Substrate I was also estimated by HPLC analysis of HIV protease/Substrate reaction mixtures at times before the hydrolysis reaction was complete. Integration of the peaks on the fluorescence and 475 nm absorbance chromatograms (Figure 2) yielded an apparent 33-fold enhancement of the fluorescence quantum yield of the Pro-Ile-Val-Gln-EDANS peptide fragment over the parent composition, Substrate I. However, a value of 40-fold enhancement that was obtained under

assay conditions is likely to be more accurate because the HPLC, although performed at a similar pH (4.9), was carried out in an acetonitrile-water solvent system. The acetonitrile-water medium alters the photophysical properties of the probes and the conformation of the peptide, which in turn affects the degree of quenching by intramolecular resonance energy transfer.

Proof that the enzymatic cleavage of the fluorogenic substrate by protease was specific and quantitative was obtained in the following manner. First, HPLC analysis of the reaction products after a 2.5 hour incubation with HIV protease indicated complete disappearance of the original peptide eluting at 53.5′ and the concomitant appearance of two new species with retention times of 36.4′ and 47.2′ (Figure 3 ). This result suggested that the fluorogenic substrate had been cleaved quantitatively at a single bond. The 36.4′ peak exhibited a fluorescence yield characteristic of the unquenched EDANS fluorophore, while the 47.2′ species was non-fluorescent and present on the 475 nm absorbance chromatogram. The absorption spectrum for each peak, acquired on-line during the chromatography, confirmed the assignment of the peaks as corresponding to EDANS (36.4′), DABCYL (47.2′), and EDANS + DABCYL (53.5′).

The cleavage products of Substrate I, isolated by HPLC, were also subjected to amino acid analysis. The fragment eluting at 36.4′ was identified as Pro-Ile-Val-Gln-EDANS. The fragment eluting at 47.2′ was identified as DABCYL-Ser-Gln-Asn-Tyr. The analysis confirmed that proteolysis of Substrate I occurred specifically at the Tyr-Pro bond. In addition, these two chromophore-labeled tetrapeptide fragments were independently synthesized, and their retention times on reverse phase HPLC were indistinguishable from the HIV protease liberated fragments predicted to be the substrate's specific cleavage products.

## Example 5

### Inhibition of HIV Protease by Pepstatin

The inhibition of HIV protease activity by pepstatin was also examined using the novel fluorogenic substrates of the present invention. Pepstatin is a potent inhibitor of many cellular aspartic proteinases and is a reported inhibitor of HIV protease [S. Seelmeier, et al., Proc. Natl. Acad. Sci. 85, 6612 (1988); C.Z. Giam, et al., J. Biol. Chem. 263,14617 (1988); J. Schneider, et al., Cell 54, 363 (1988); J. Hansen, et al., EMBO J. 7, 1785 (1988); P.L. Darke, et al., J. Biol. Chem. 264, 2307 (1989); A.D. Richards, et al., FEBS Lett 247, 113 (1989); R.F. Nutt, et al., Proc. Natl. Acad. Sci. 85, 7129 (1988); H.G. Krausslich, et al., Proc. Natl. Acad. Sci. 86, 807 (1989)].

This example illustrates the measurement of the inhibition of HIV protease by pepstatin at pH 4.7, 30 ° C. The reaction was performed substantially in accordance with the procedure described in Example 2.

The data were obtained for three concentrations of Substrate I (10 µM, 33 µM and 129 µM) and were plotted in the form of a Dixon plot of pepstatin concentration (nM) vs. 1/V (reaction velocity; min/nM) [M. Dixon, Biochem. J. 55, 170 (1953) which is incorporated by reference herein.] The data are presented in Figure 4. The point of intersection of the three lines yields an inhibitory constant ($K_i$) of 17 nanomolar which indicates that pepstatin is a very potent inhibitor of HIV protease, as it is for most aspartic proteases.

Data plotted according to the method of Cornish-Bowden, pepstatin concentration (nM) vs. substrate concentration/V (min/nM), in Figure 5 [A. Cornish-Bowden, Biochem. J. 137, 143 (1974)] produced parallel lines, demonstrating that inhibition of the protease by pepstatin was purely competitive. (The data for the 33 µM Substrate I concentration were nearly coincident with the 10 µM data and were omitted for clarity.)

## Example 6

### Fluorogenic Assay for AMV Protease

Fluorogenic Substrate II (DABCYL-gaba-Ser-Val-Val-Tyr-Pro-Val-Val-Gln-EDANS) was found to be an efficient substrate for AMV protease, and should therefore be useful for identifying and characterizing inhibitors for this enzyme. The assay was performed substantially in accordance with the procedure

described in Example 3.

AMV protease (Molecular Genetic Resources, Tampa, FL) was at least 50-fold more active against Substrate II than against Substrate I. Conversely, HIV protease cleaved Substrate I (3.4 μM) ten times faster than Substrate II (3.4 μM). Pepstatin inhibited AMV protease fairly weakly in this assay, with an $IC_{50}$ - (concentration of inhibitor yielding 50% inhibition) of about 40 micromoles (μM) at pH 6.

## Example 7

## Preparation of Different Fluorogenic Substrates

The choice of both the fluorophore and the chromogenic quencher was key to the successful design of the viral protease fluorophore-quencher substrates of the present invention. Optimal spectral overlap, i.e., overlap of the emission of the fluorescent donor with the absorption of the quenching acceptor, was desired to obtain the lowest background fluorescence and therefore the greatest dynamic range for the assay. Consideration was also given to the solubility of the acceptor and donor in aqueous buffer. DABCYL was chosen as the quencher and EDANS as the fluorophore based upon these considerations.

A series of peptides corresponding to various cleavage sites of HIV protease and AMV protease were functionalized with a DABCYL group at the N-terminus and EDANS at the C-terminus to form a series of fluorophore-quencher protease substrates. The substrates were made substantially in accordance with the procedure described above in Example 1. In this procedure it was found that the azo-type chromophore was attached to the N-terminal of peptides via an acetyl 4-amino-butyric acid linkage.

Alternatively, a sulfamide of a 4-methylamino butyric acid (maba) linkage could be used. The corresponding N-labelling reagent, DABSYL-maba-NHS, was prepared as follows.

a. Preparation of N-methyl-N-[4-(4′-dimethylaminophenyl)azo-benzenesulfonyl]-r-amino butyric acid. (DABSYL-maba-OH)

r-Methylamino butyric acid (1.0 g, excess, Aldrich Chemical Co.) was dissolved in water (50 mL), and the solution was adjusted to pH 10 with sodium carbonate. A solution of 4-(4′-dimethylaminophenyl)azo-benzenesulfonyl chloride (0.85 g, 2.63 mmol, Sigma) in dimethoxyethane (30 mL) was added. The mixture was refluxed overnight. After cooling, the mixture was concentrated and the residue was chromatographed on a silica gel column with 20% MeOH/EtOAc as the solvent to yield a red solid product (0.75 mg).

b. Preparation of N-methyl-N-[4-(4′-dimethylaminophenyl)azobenzenesulfonyl]-r-amino butyric acid N-hydroxy succinimide. (DABSYL-maba-NHS)

N-methyl-N-[4-(4′-dimethylaminophenyl)azo-benzene-sulfonyl]-r-amino butyric acid (DABSYL-maba-OH, 0.75 g, 1.9 mmole) was suspended in dry dimethoxyethane (50 mL). N-hydroxy succinimide (0.3 g, 2.8 mmole)) and dicyclohexylcarbodiimide (0.45 g, 2.2 mmole) were added. After completion of the reaction, as indicated by TLC, the mixture was filtered to remove DCU by-product. The filtrate was concentrated to dryness, and the residue was triturated with methylene chloride twice. The solution was then chromatographed on a silica gel column with EtOAc as solvent to give the desired product DABSYL-maba-NHS (0.90 g).

The peptide sequence and fluorescent donor were then attached substantially in accordance with the procedure described in Example 1, above.

The different fluorogenic protease substrates, which were prepared according to the procedures described in Examples 1 and 7, are listed in Table 1.

Table 1

| Fluorogenic Substrates and Protease Activity | | |
|---|---|---|
| Substrate | HIV protease Activity | AMV protease Activity |
| I DABCYL-gaba-Ser-Gln-Asn-Tyr-Pro-Ile-Val-Gln-EDANS | + | - |
| II DABCYL-gaba-Ser-Val-Val-Tyr-Pro-Val-Val-Gln-EDANS | - | + |
| III DABSYL-maba-Val-Ser-Phe-Asn-Phe-Pro-Gln-Ile-Thr-Leu-EDANS | NT* | NT* |
| IV DABSYL-maba-Ser-Gln-Asn-Tyr-Met-Ile-Val-Gln-EDANS | NT* | NT* |
| V DABCYL-gaba-Ile-Arg-Gln-Ala-Asn-Phe-Leu-Gly-Leu-EDANS | + | NT |
| VI DABCYL-gaba-Ala-Thr-Leu-Asn-Phe-Pro-Ile-Ser-Gln-Glu-EDANS | + | NT |
| VII DABCYL-gaba-Thr-Ala-Thr-Ile-Met-Met-Gln-Arg-Gly-Glu-EDANS | + | NT |
| VIII DABCYL-gaba-Thr-Phe-Gln-Ala-Tyr-Pro-Leu-Arg-Gln-Ala-EDANS | - | + |

*The compound was insufficiently soluble in the assay buffer used.
NT = not tested.

Example 8

Protease Activity Towards Fluorogenic Substrates

The activity of HIV protease and AMV protease towards the different fluorogenic substrates was tested substantially in accordance with the procedure described in Example 3 above. The results are summarized in Table I. Among these compounds, DABCYL-gaba-Ser-Gln-Asn-Tyr-Pro-Ile-Val-Gln-EDANS was studied most thoroughly. This compound was' found to be cleaved quite efficiently by HIV protease, but not by AMV protease. It was cleaved specifically at the Tyr-Pro linkage to give a fluorescence signal which allowed continuous monitoring of enzymatic activity. Enzyme kinetics determinations indicated a Michaelis-Menten kinetics constant ($K_m$) of 103 $\mu$M and a maximum velocity for the enzymatic reaction ($V_{max}$) of 47 nM/minute. The enzymatic activity of the other compounds in Table I was determined only qualitatively as indicated. Substrates V and VI were cleaved by HIV protease, but not as efficiently as was Substrate I. Substrate VII was found to be a more efficient substrate the Substrate I, with a $K_m$ = 5.9 $\mu$M and $V_{max}$ = 1.8 nM/minute.

Example 9

Fluorogenic Donor/Acceptor Substrates with Improved Water Solubility

Although it was anticipated that the negative charge of EDANS would afford the DABCYL/EDANS, donor/acceptor substrates the desired solubility in aqueous media, solubility was a limiting factor. The enzyme activity of compounds 3 and 4, from Table 1, was not determined due to their poor solubility.

Two approaches were taken to increase the solubility of the substrates: modification of the donor or acceptor group, or modification of the peptide.

In the first approach, fluorophores or chromophores with more charges were used to replace EDANS or DABCYL. Toward this end, a lucifer yellow type fluorophore, N-(2-aminopentyl)-3-amino-2,7-disulfo-1,8-naphthalimide dipotassium salt was used. The two ionic sulfonic acid groups in this molecule were to provide the desired enhancement in solubility.

Two compounds were prepared with this fluorophore, DABCYL-gaba-Ser-Gln-Asn-Tyr-Pro-Ile-Val-Gln-lucifer yellow type fluorophore and DABCYL-gaba-Val-Ser-Phe-Asn-Phe-Pro-Gln-Ile-Thr-Leu-lucifer yellow type fluorophore. The substrates were prepared substantially in accordance with the procedure described in Example 1.

Assays, performed substantially in accordance with the procedure described in Example 3, have shown that both compounds are HIV protease active. Data indicated an upper limit of solubility for DABCYL-gaba-Ser-Gln-Asn-Tyr-Pro-Ile-Val-Gln-lucifer yellow type fluorophore in pH 4.7 NaOAc buffer of around 350 μM or higher, in comparison with a solubility of about 150 μM for the corresponding EDANS substrate.

In pH 4.7 buffer, the lucifer yellow compound showed an excitation maximum at 440 nm and emission maximum at 520, whereas the absorption maximum of DABCYL was around 470 nm at pH 4.7. Thus, for optimal protease substrate preparations, a chromogenic quencher having a more optimal spectral overlap with the lucifer yellow type fluorophore should be used to replace DABCYL acceptor group.

The second approach to enhance solubility was to modify the peptide sequence, by adding polar amino acids at the terminal furthest from the cleavage site. Examples include His-Ser-Gln-Asn-Tyr-Pro-Ile-Val-Gln-His, in which the histidines boost the solubility, or Arg-Val-Ser-Phe-Asn-Phe-Pro-Gln-Ile-Thr-Arg, in which arginines serve to enhance the solubility. The substrates which included these peptide structures were made substantially in accordance with the procedure described in Example 1

It will be appreciated by one skilled in the art that the concepts of the present invention are equally applicable to the production of fluorogenic substrates for other enzymes, as well as for the use of such substrates in a variety of fluorescent assay procedures or the production of reagent or assay kits. The present invention teaches the production of a fluorogenic substrate, using the intramolecular resonance energy transfer between a fluorescent donor and a quenching acceptor, for use in detecting the presence of a viral protease enzyme The embodiments described herein are intended as examples rather than as limitations and are intended to encompass all equivalents and subject matter within the spirit and scope of the invention as described above and as set forth in the following claims.

## Claims

1. A fluorogenic substrate, comprising:
a. a peptide,
b. a fluorescent donor attached to said peptide, and
c. a quenching acceptor attached to said peptide,
wherein said peptide has a configuration sufficient for intramolecular resonance energy transfer between said fluorescent donor and said quenching acceptor, and wherein said peptide has a viral protease enzyme-cleavable site.

2. The fluorogenic substrate according to Claim 1, wherein said fluorescent donor and said quenching acceptor are separated at a distance of less than about 100 angstroms.

3. The fluorogenic substrate according to Claim 1, wherein said fluorescent donor is selected from the group consisting of: fluorescein and fluorescein derivatives; N-(2-aminopentyl)-3-amino-2,7-disulfo-1,8-naphthalimidedipotassium salt and derivatives thereof; 5-((2-aminoethyl)amino)naphthalene-l-sulfonic acid; and coumarin and coumarin derivatives.

4. The fluorogenic substrate according to Claim 1, wherein said quenching acceptor is selected from the group consisting of 2,4-dinitrophenyl; 4-(4-dimethyldaminophenyl) azobenzene sulfonic acid; and 4-(4-dimethylaminophenyl) azobenzoic acid and derivatives thereof.

5. The fluorogenic substrate according to Claim 1, wherein said peptide is selected from the group consisting of:
Ser-Gln-Asn-Tyr-Pro-Ile-Val-Gln;
Ser-Val-Val-Tyr-Pro-Val-Val-Gln;
Val-Ser-Phe-Asn-Phe-Pro-Gln-Ile-Thr-Leu;
Ser-Gln-Asn-Tyr-Met-Ile-Val-Gln;
Ile-Arg-Gln-Ala-Asn-Phe-Leu-Gly-Leu;
Ala-Thr-Leu-Asn-Phe-Pro-Ile-Ser-Gln-Glu;
Thr-Ala-Thr-Ile-Met-Met-Gln-Arg-Gly-Glu;
Thr-Phe-Gln-Ala-Tyr-Pro-Leu-Arg-Gln-Ala;
His-Ser-Gln-Asn-Tyr-Pro-Ile-Val-Gln-His; and
Arg-Val-Ser-Phe-Asn-Phe-Pro-Gln-Ile-Thr-Arg.

6. The fluorogenic substrate according to Claim 1, having either the formula:

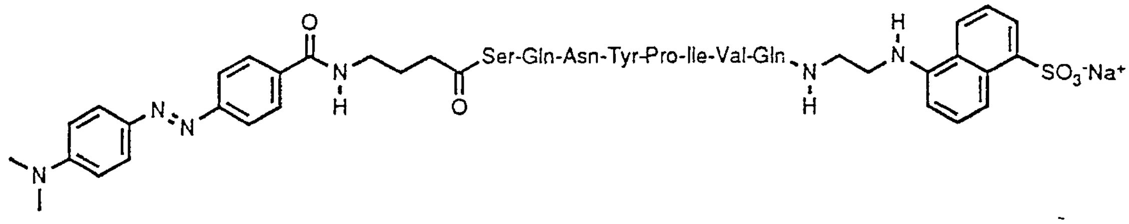

DABCYL EDANS

or the formula:

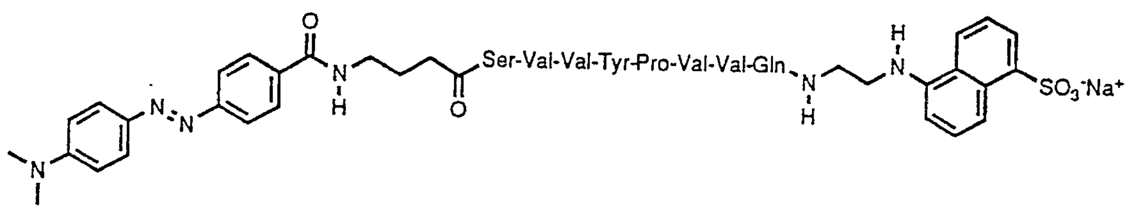

DABCYL EDANS

7. The fluorogenic substrate according to Claim 1, wherein said fluorescent donor is attached to said peptide's C-terminus and said quenching acceptor is attached to said peptide's N-terminus.

8. The fluorogenic substrate according to Claim 1, wherein said viral protease enzyme is human immunodeficiency virus protease.

9. The fluorogenic substrate according to Claim 1, wherein said viral protease enzyme is avian myeloblastosis virus protease.

10. An assay for detecting the presence or activity of a viral protease enzyme in a test sample, comprising the steps of:

a. combining the test sample with a fluorogenic substrate, wherein said fluorogenic substrate comprises

i. a peptide,

ii. a fluorescent donor attached to said peptide, and

iii. a quenching acceptor attached to said peptide, wherein said peptide has a configuration sufficient for intramolecular resonance energy transfer between said fluorescent donor and said quenching acceptor, and wherein the enzyme cleaves said substrate, thereby separating said donor and said acceptor; and

b. detecting said donor's fluorescent emission.

11. The assay according to Claim 10, further comprising continuously detecting said donor's fluorescent emission due to substrate hydrolysis.

12. The assay according to Claim 10, wherein the protease enzyme is human immunodeficiency virus protease, and wherein said fluorogenic substrate has the formula:

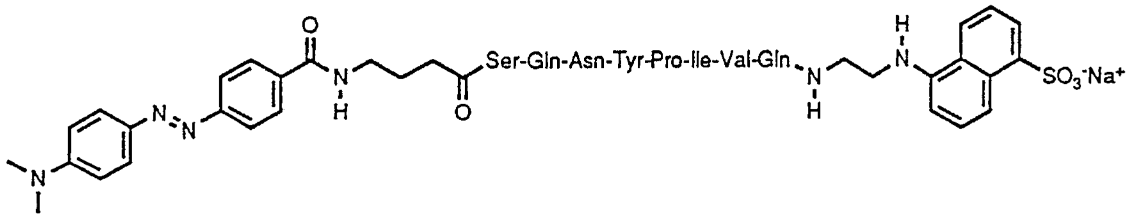

DABCYL EDANS

13. The assay according to Claim 10, wherein the protease enzyme is avian myeloblastosis virus protease and wherein said fluorogenic substrate has the formula:

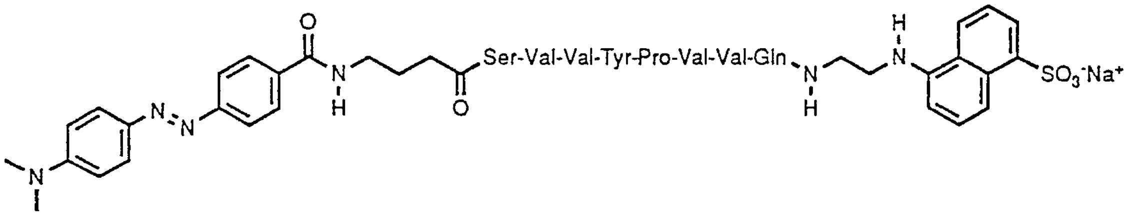

DABCYL EDANS

14. An assay for detecting the activity of a protease enzyme inhibitor, comprising the steps of:

a. combining the inhibitor with a protease enzyme and a fluorogenic substrate, wherein said fluorogenic substrate comprises

i. a peptide,

ii. a fluorescent donor attached to said peptide, and

iii. a quenching acceptor attached to said peptide, wherein said peptide has a configuration sufficient for intramolecular resonance energy transfer between said fluorescent donor and said quenching acceptor, and wherein said enzyme cleaves said substrate, thereby separating said donor and said acceptor; and

b. detecting said donor's fluorescent emission.

15. The assay according to Claim 14, further comprising continuously detecting said donor's fluorescent emission due to substrate hydrolysis.

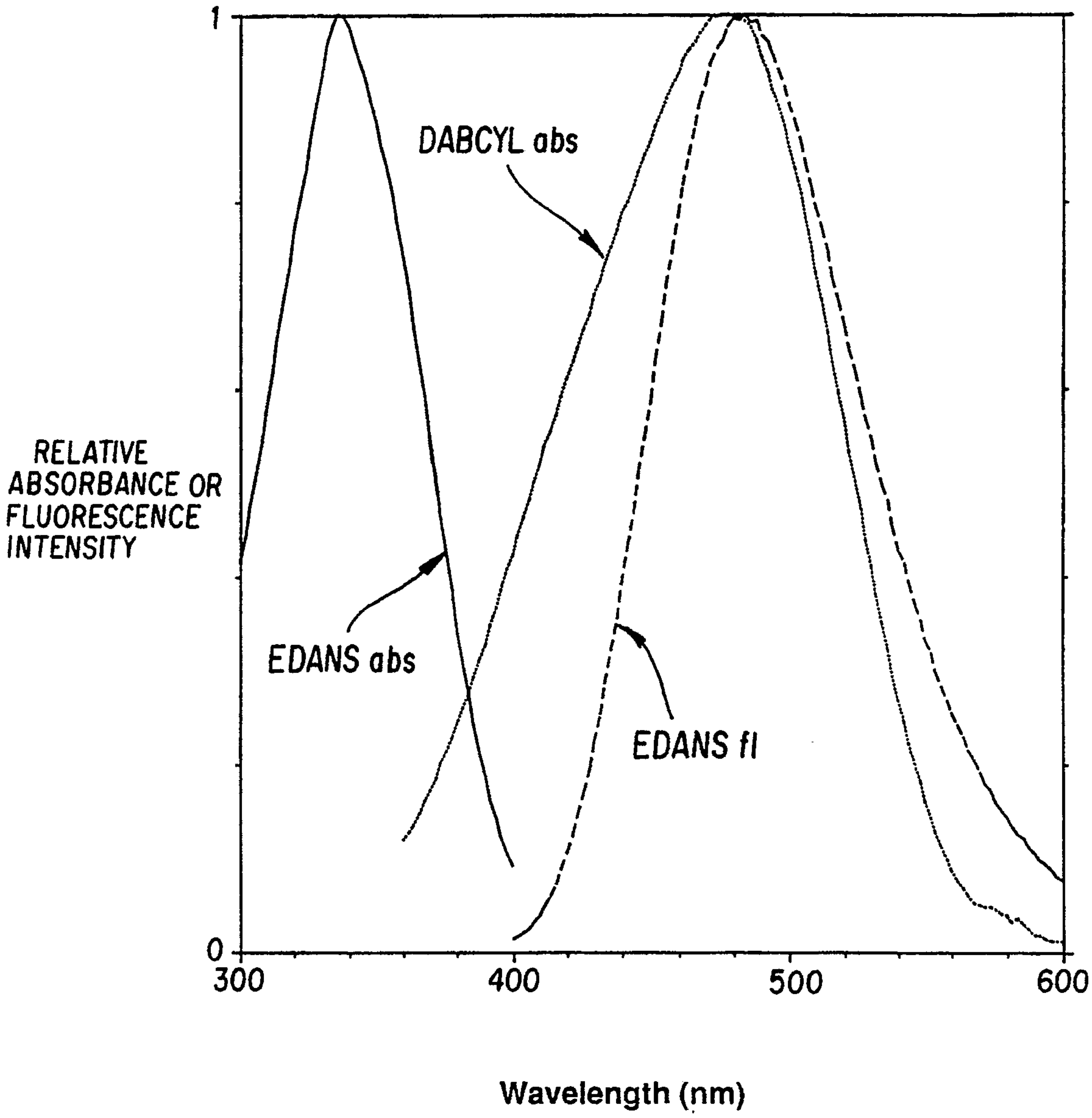
1
0
300
400
500
600
DABCYL abs
EDANS abs
EDANS fl
RELATIVE ABSORBANCE OR FLUORESCENCE INTENSITY
Wavelength (nm)

FIG. 1

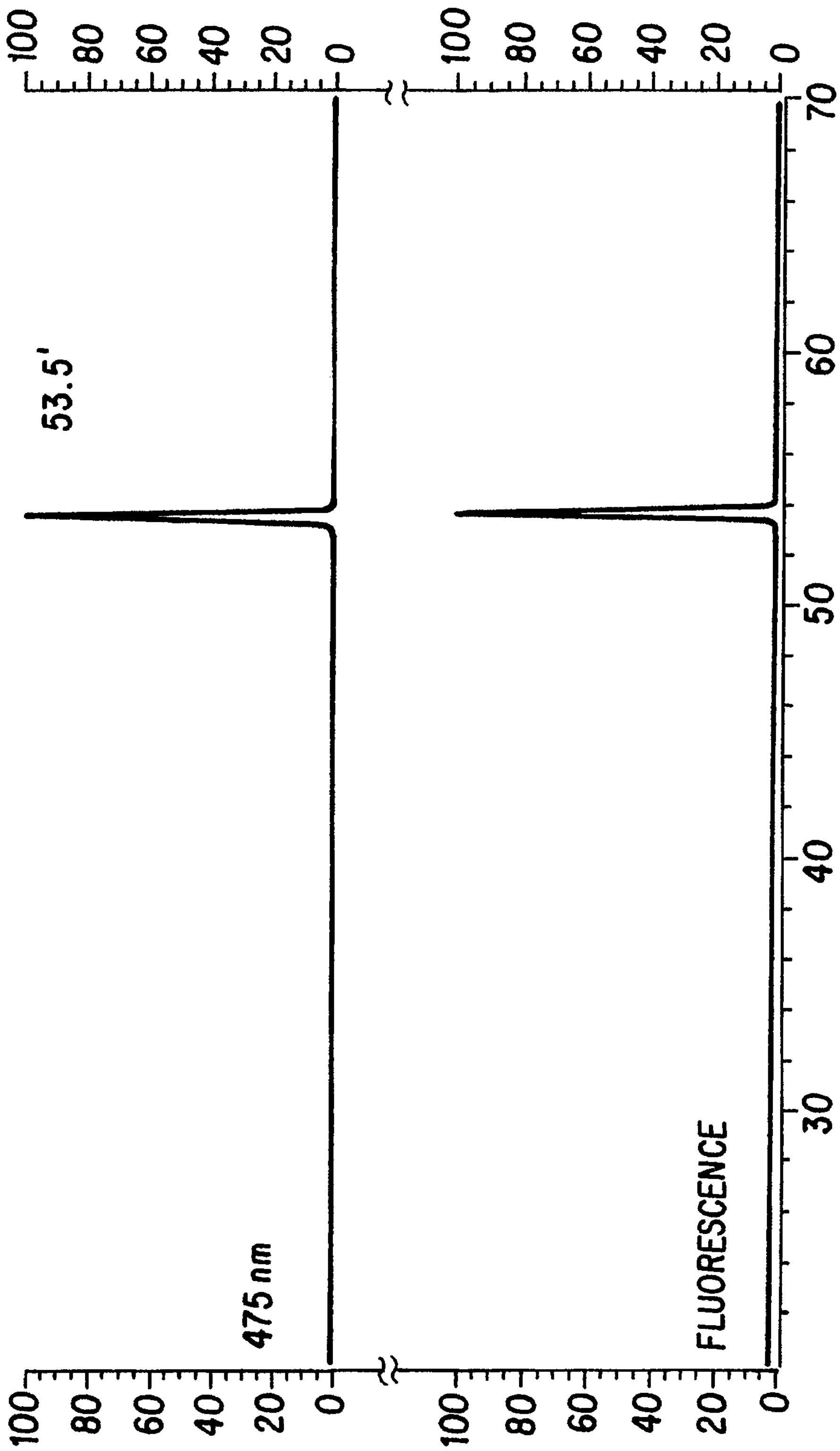


FIG. 2

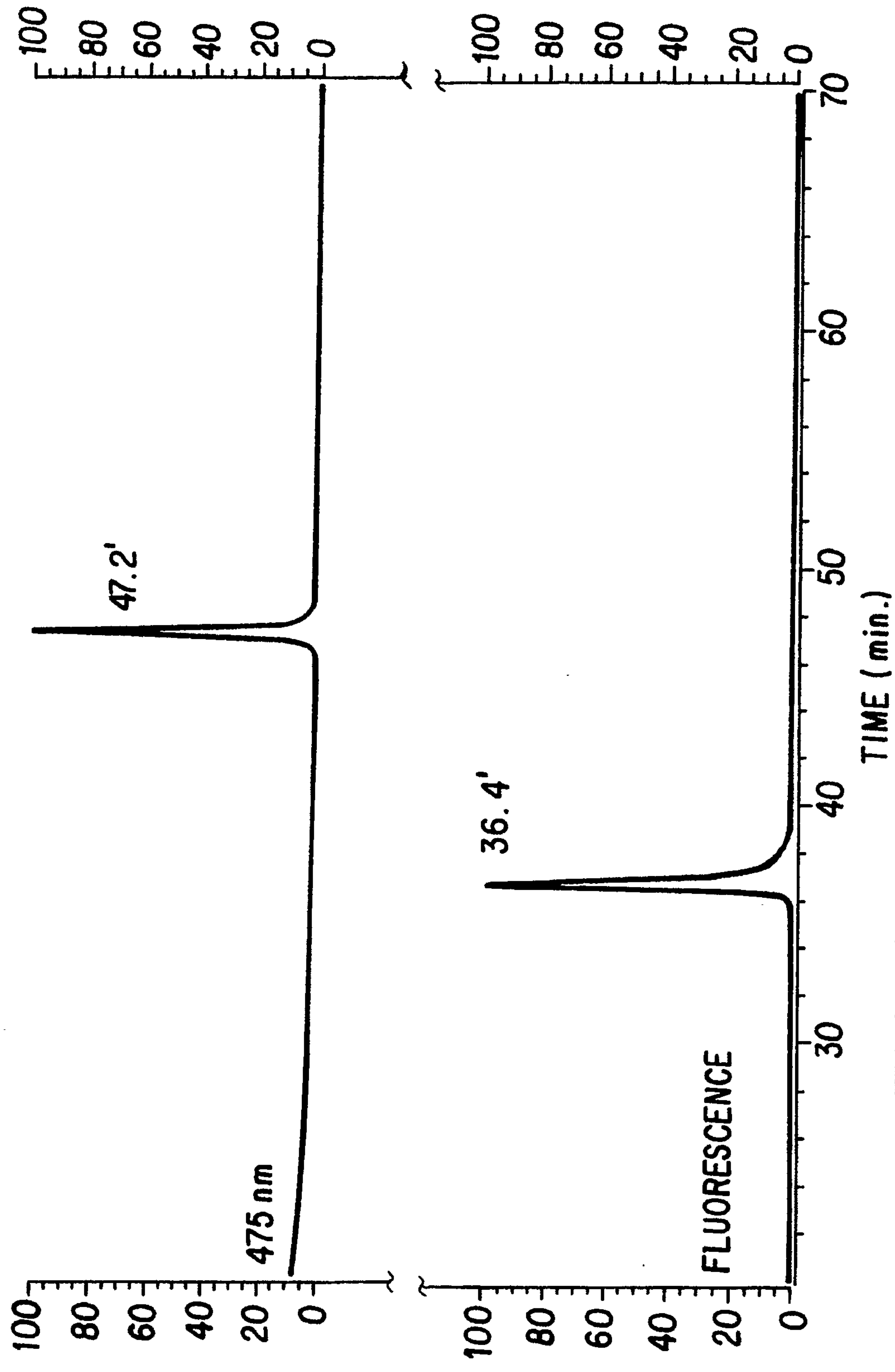
475 nm
47.2'
36.4'
FLUORESCENCE
TIME (min.)
30
40
50
60
70
100
80
60
40
20
0

FIG. 3

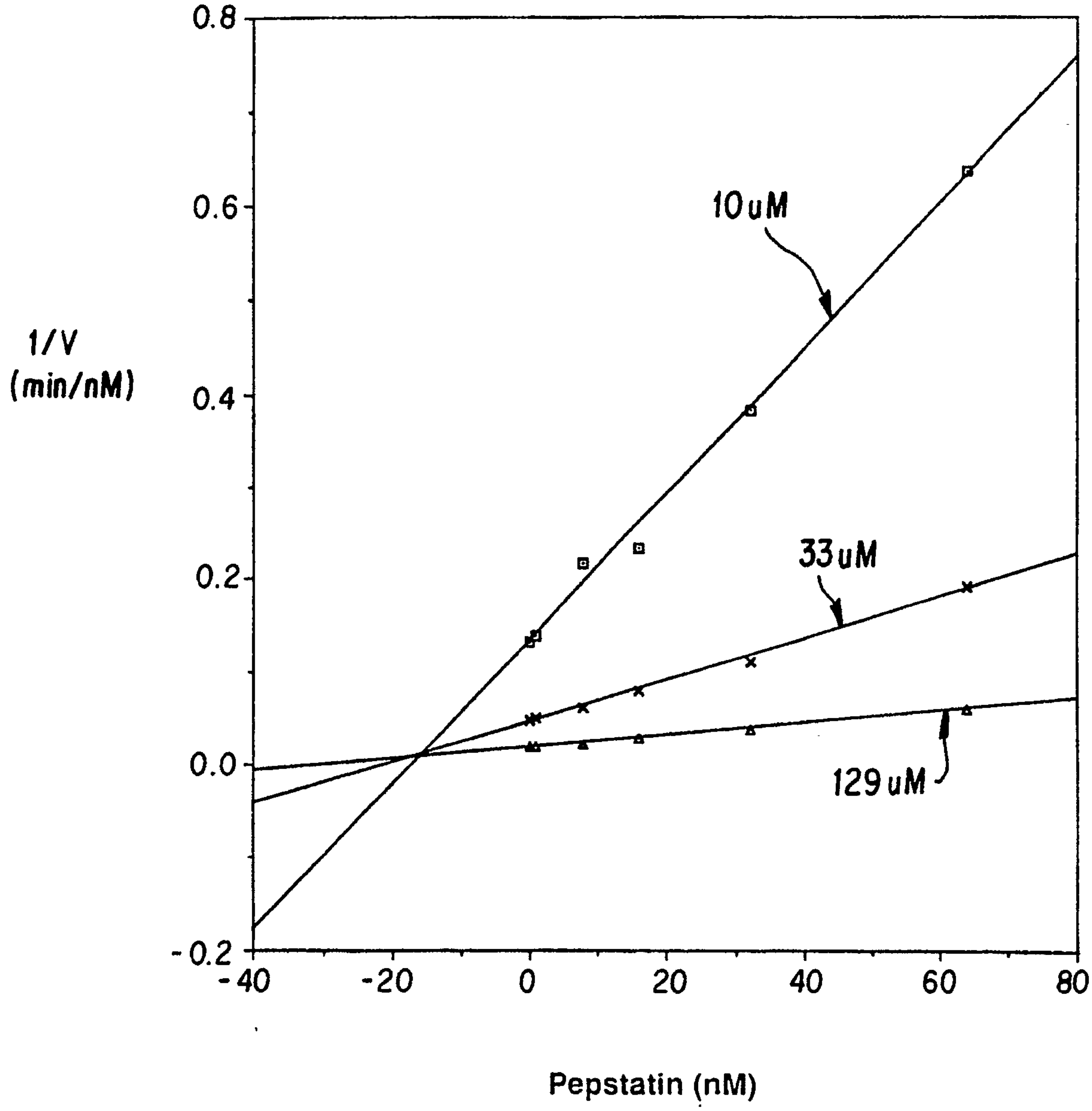
0.8
0.6
0.4
0.2
0.0
- 0.2
1/V
(min/nM)
10uM
33uM
129uM
- 40
-20
0
20
40
60
80
Pepstatin (nM)

FIG. 4

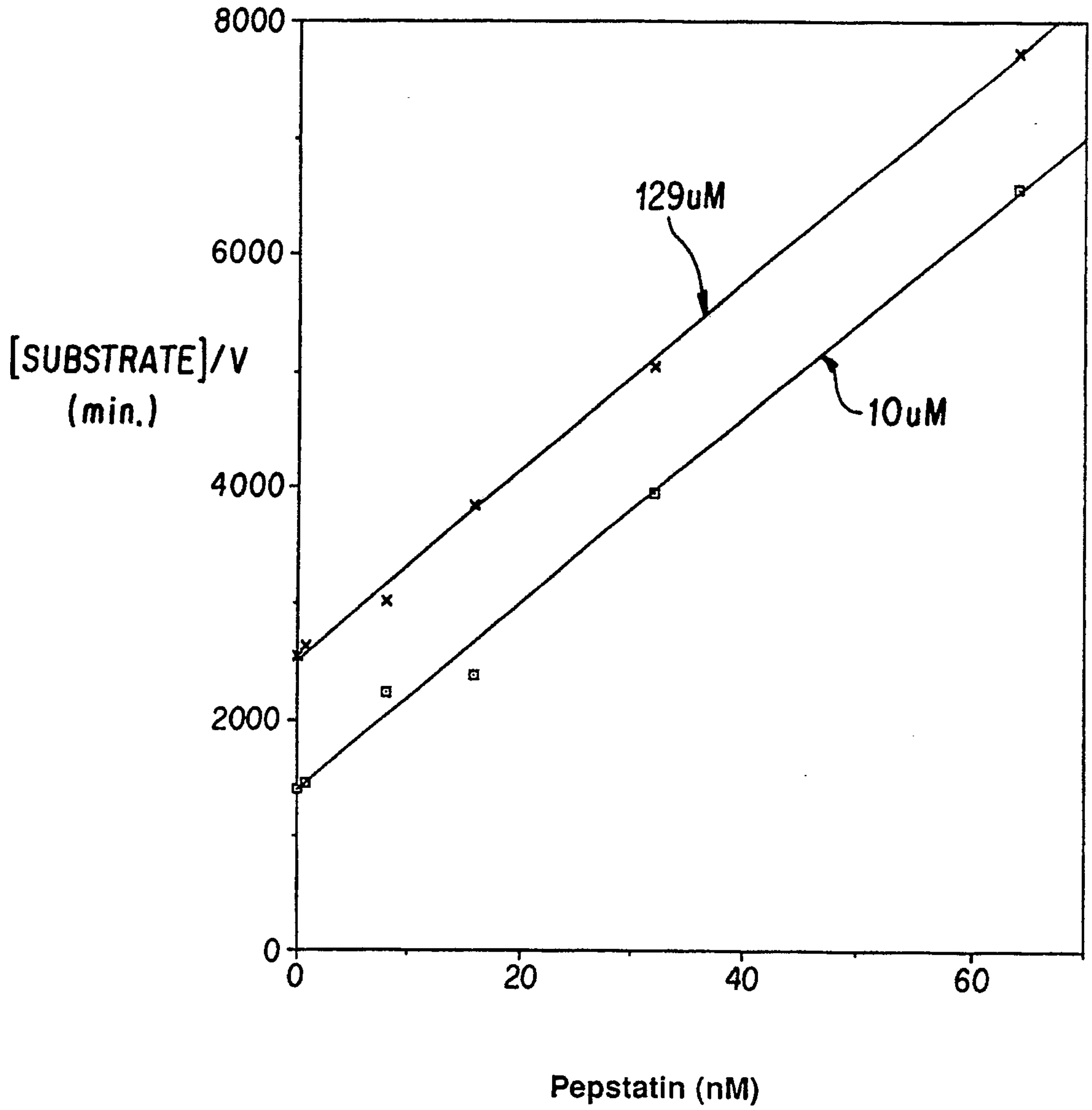
8000
6000
4000
2000
0
0
20
40
60
[SUBSTRATE]/V
(min.)
129uM
10uM
Pepstatin (nM)

FIG.5

European Patent Office

# EUROPEAN SEARCH REPORT

Application Number

**EP 90 12 0724**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 822 746 (D.R. WALT)<br>* Figure 5; abstract *<br>– – – | 1 | C 12 Q 1/37<br>G 01 N 33/533<br>G 01 N 33/58<br>C 07 K 7/06 |
| Y | EP-A-0 373 576 (HOECHST AG)<br>* The entire document *<br>– – – | 1 | |
| A | | 5,6,10-15 | |
| A | EP-A-0 229 943 (MOLECULAR BIOSYSTEMS INC.)<br>* Abstract; page 1, line 13 - page 5, line 4; figure 1 *<br>– – – | 1-15 | |
| A | GB-A-2 223 096 (UNITED KINGDOM ATOMIC ENERGY AUTHORITY)<br>* The entire document *<br>– – – | 1-15 | |
| A | EP-A-0 046 742 (KABI VITRUM AB)<br>* The entire document *<br>– – – | 1-15 | |
| D,A | THE JOURNAL OF BIOLOGICAL CHEMISTRY, vol. 255, no. 8, 25th April 1980, pages 3482-3486, Baltimore, MD, US; N. NISHINO et al.: "Pseudomonas aeruginosa elastase"<br>* The entire document *<br>– – – | | TECHNICAL FIELDS SEARCHED (Int. Cl.5) |
| D,A | THE JOURNAL OF THE AMERICAN CHEMICAL SOCIETY, vol. 111, no. 15, 19th July 1989, pages 5961-5962; S.J. POLLACK et al.: "Stereospecific hydrolysis of alkyl esters by antibodies"<br>* Page 5961, right-hand column, lines 14-19 *<br>– – – – – | | C 12 Q<br>G 01 N<br>C 07 K |

The present search report has been drawn up for all claims

| Place of search | Date of completion of search | Examiner |
|---|---|---|
| The Hague | 13 February 91 | DOEPFER K-P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document oited for other reasons

&: member of the same patent family, corresponding document